# EUROPEAN PATENT APPLICATION

(11) **EP 2 601 967 A1**
(43) Date of publication of application: **12.06.2013**
(21) Application number: 11814861.8
(22) Date of filing: 15.07.2011
(51) Int. Cl.: A61K 39/00, A61P 31/18

(54) **DRUG AND METHOD FOR THE PROPHYLAXIS OF HIV INFECTION AND FOR THE PROPHYLAXIS AND TREATMENT OF DISEASES CAUSED BY OR ASSOCIATED WITH HIV, INCLUDING AIDS**

(30) Priority: 06.08.2010 RU 2010133045
(71) Applicant: Epshtein, Oleg Iliich, Moscow 127473 (RU)
(72) Inventor: TARASOV, Sergei Alexandrovich, Golytsino Moskovskaya obl. 143040 (RU); EPSHTEIN, Oleg, Iliich, Moscow 127473 (RU)
(74) Representative: Müller-Boré & Partner Patentanwälte
(86) International application number: PCT/RU2011/000523
(87) International publication number: WO 2012/018284

(57) **Abstract**

The medicinal agent for the prevention of HIV contamination, prevention and treatment of the diseases caused by HIV or HIV-associated diseases including AIDS contains activated-potentiated form of antibodies to the protein or peptide of the immune system which interacts with HIV or its content and/or functional activity changes in relation with the contamination of HIV. Meanwhile, in the prevention method of HIV contamination, prevention and treatment of the diseases caused by HIV or HIV-associated diseases, including AIDS, the activated-potentiated form of antibodies to antigen - protein or peptide of the immune system, which interacts with HIV or its content and/or functional activity changes in relation to HIV contamination, is used.

## Description

### Pertinent art

The invention belongs to the field of medicine and can be used for the effective prevention of HIV contamination, prevention and treatment of diseases caused by HIV and HIV-associated diseases including AIDS.

### Prior art

The prior art includes a medicinal agent for treatment of infectious diseases including viral diseases based on the activated form of very-low-dose anti-interferon antibodies (RU 2192888 C1, A61K39/395, 20.11.2002). However, this medicinal agent may neither be effective for the prevention of HIV contamination nor for the prevention and treatment of a wide range of diseases caused by HIV or HIV-associated diseases including AIDS.

### Disclosure of the invention

The invention is directed to the development of a complex medicinal agent without marked side effects allowing the effective prevention of HIV contamination as well as the prevention and effective treatment of diseases caused by HIV and HIV-associated diseases including infections and infestations, malignant neoplasms, and AIDS in HIV positive subjects.

The solution of this problem is assured by the fact that the medicinal agent for the prevention of HIV contamination and prevention and treatment of diseases caused by HIV and HIV-associated diseases including AIDS, according to the invention, contains an activated-potentiated form of antibodies to antigen - a protein or a peptide of the immune system or, predominantly, produced by the immune system which reacts with HIV or the content or the functional activity of which are altered in the presence of HIV.

At that, the activated-potentiated form of antibodies directed predominantly to the solved antigen (or soluble antigen i.e. the antigen not bound to the outer membrane of immune cells) can be used.

Cytokines (except for gamma-interferon) can be used as soluble antigens.

Besides that, an activated-potentiated form of antibodies directed predominantly to the antigen bound to the outer membrane of the immune cells can also be used.

At that, immunocompetent cell receptors are used as an antigen bound to the outer membrane of immune cells.

Furthermore, clusters of differentiation (except for CD4 molecule of T-lymphocytes) can also be used as antigens bound to the outer membrane of the immune cells.

The activated-potentiated form of antibodies to solved antigens or the activated-potentiated form of antibodies to antigens bound to the outer membrane of the immune cells are used in the form of activated-potentiated aqueous or aqueous-alcoholic solution the activity of which is based on the process of serial multiple dilution of the stock (initial) solution of antibodies in aqueous or aqueous-alcoholic solvent combined with the external mechanical impact - vertical shaking after each dilution.

At that, the claimed medicinal agent can be designed in solid dosage form as a pharmaceutical composition that contains technologically required (effective) amount of neutral vehicle saturated with mixture of aqueous and aqueous-alcoholic solutions of the activated-potentiated form of antibodies to solved antigens or the activated-potentiated form of antibodies to antigens bound to the outer membrane of the immune cells and pharmaceutically acceptable excipients including, for example, lactose, microcrystal cellulose, and magnesium stearate.

Aqueous and aqueous-alcoholic solutions of the activated-potentiated forms of antibodies to solved antigens or antigens bound to the outer membrane of the immune cells can be obtained by serial multiple dilution of the stock (initial) solution of antibodies combined with the external mechanical impact - vertical shaking after each dilution; the stock solution concentration is 0.5 ÷ 5.0 mg/mL.

Activated-potentiated form of the antibodies can be used in the form of mixture of different, predominantly centesimal, dilutions by the homeopathic technology.

The solution of this problem is also assured by the fact that in the mode of prevention of HIV contamination and prevention and treatment of diseases caused by HIV or HIV-associated diseases including AIDS used in the invention, the activated-potentiated form of antibodies to antigen (protein or peptide of the immune system or, predominantly, produced by the immune system) which reacts with HIV or the content or the functional activity of which is altered by HIV contamination.

The activated-potentiated form of antibodies to solved antigens or the activated-potentiated form of antibodies to antigens bound to the outer membrane of the immune cells are used in the form of activated-potentiated aqueous or aqueous-alcoholic solution of each component the activity of which is based on the process of multiple dilution of the stock (initial) solution of antibodies in aqueous or aqueous-alcoholic solvent combined with the external mechanical impact - vertical shaking after each dilution.

Aqueous and aqueous-alcoholic solutions of the activated-potentiated forms of antibodies to solved antigens or antigens bound to the outer membrane of the immune cells are predominantly obtained by serial multiple dilution of the stock (initial) solution of antibodies combined with the external mechanical impact - vertical shaking after each dilution; the stock solution concentration is 0.5 ÷ 5.0 mg/mL.

According to the invention, the activated-potentiated form is a form of antibodies prepared in accordance with homeopathic technology of potetiation by the serial multiple dilution of the stock (initial) solution of antibodies combined with the external mechanical impact - vertical shaking after each dilution which is active in pharmacological models and/or clinical methods of prevention of HIV contamination and prevention and treatment of diseases caused by HIV or HIV-associated diseases including AIDS.

Proposed use of the activated-potentiated form of antibodies to solved antigens (for example, to tumor necrosis factor alpha or human alpha interferon) or antigens bound to the outer membrane of the immune cells (for example, to CD8 receptor) leads to the unexpected therapeutic effect that consists in higher efficacy of the medicinal agent in prevention of HIV contamination as well as in prevention and treatment of diseases caused by HIV or HIV-associated diseases including AIDS.

It is shown experimentally that the claimed medicinal agent is characterized by high preventive efficacy towards HIV preventing the penetration of human immunodeficiency virus into the cells and its intracellular replication and, thus, it can be used for the effective treatment as well as for the prevention of viral diseases tended to chronic course including secondary prevention of HIV-infection.

The claimed medicinal agent may be used in combination with antiretroviral agents including complex agents such as reverse transcriptase inhibitors (for example, zidovudine derivatives) which allows a reduction of dose of antiretroviral agents in constant high efficacy of the therapy and decreases the rate of adverse events.

### Embodiments of invention

The medicinal agent is prepared mainly in the following way.

For preparation of the activated-potentiated form of active substances, monoclonal or (predominantly) polyclonal antibodies are used; they may be obtained using known technologies, particularly, techniques described, for example, in Immunological methods, under the editorship of G. Frimel, M, 'Meditsina', 1987, p.9-33 [Russian*];* or in the article Laffly E., Sodoyer R. Hum. Antibodies. Monoclonal and recombinant antibodies, 30 years after. - 2005 - Vol. 14. - N 1-2. P. 33-55*.*

Monoclonal antibodies are obtained, for example, using hybridoma technology. At that, the initial stage of the process includes immunization based on principles that has already been developed for polyclonal antiserum preparation. Further stages of this process involve obtaining of hybridoma cells producing clones of antibodies of the same specificity. Their separation is performed using the same methods as for polyclonal antisera.

Polyclonal antibodies can be obtained by active immunization of animals. For this purpose, using a special scheme, a series of injections of the substance required according to the invention - antigen or conjugated antigen (protein or peptide of the immune system or, predominantly, produced by the immune system which reacts with HIV or the content and/or the functional activity of which are altered by HIV contamination) are performed to the animals. As a result of this procedure a monospecific antiserum is obtained which is used for producing the activated-potentiated form. If necessary, a purification of antibodies present in the antiserum is performed, for example, using methods of affinity chromatography, salt fractionation, or ion-exchange chromatography.

For example, for production of the claimed medicinal agent, polyclonal antibodies to tumor necrosis factor alpha can be used, which are used as a stock (initial) solution (concentration 0.5 ÷ 5.0 mg/mL) for further preparation of the activated-potentiated form.

It is preferable to use polyclonal antibodies for preparation of the claimed medicinal agent; they can be obtained using immunization of rabbits as follows.

For example, polyclonal antibodies to tumor necrosis factor alpha (TNF-α) can be obtained using whole molecules of TNF-α with the following sequence: 
1 MSTESMIRDV ELAEEALPKK TGGPQGSRRC LFLSLFSFLI VAGATTLFCL
LHFGVIGPQR
 
61 EEFPRDLSLI SPLAQAVRSS SRTPSDKPVA HVVANPQAEG QLQWLNRRAN
ALLANGVELR
 
121 DNQLVVPSEG LYLIYSQVLF KGQGCPSTHV LLTHTISRIA VSYQTKVNLL
SAIKSPCQRE
 181 TPEGAEAKPW YEPIYLGGVF QLEKGDRLSA EINRPDYLDF AESGQVYFGIIAL

The usage of the polypeptide fragment tumor necrosis factor selected, for example, from the following consequences is possible for obtaining of polyclonal antibodies for the tumor necrosis factor - alpha (FTN - α):
84-88:
   PSDKP
93-97:
   VANPQ
65-199: 
RDLSLI SPLAQAVRSS SRTPSDKPVA HVVANPQAEG
QLQWLNRRAN ALLANGVELR DNQLVVPSEG LYLIYSQVLF
KGQGCPSTHV LLTHTISRIA VSYQTKVNLL SAIKSPCQRE
TPEGAEAKPW YEPIYLGGV
77-93:
   RSS SRTPSDKPVA HVV
32-54:
   IGPQR EEFPRDLSLI SPL
123-160:
   QLVVPSEG LYLIYSQVLF KGQGCPSTHV LLTHISRIA
176-190:
   PCQRE TPEGAEAKPW
5-45:
   SMIRDV ELAEEALPKK TGGPQGSRRC LFLSL
150-184:
   V LLTHTISRIA VSYQTKVNLL SAIKSPCQRE TPEG
77-233: 



.
 7-9 days previously to the blood sampling 1-3 intravenous injections are executed in order to increase the level of the antibodies. During the process of immunization, small amounts of blood probes are accumulated from the rabbits in order to estimate the amount of the antibodies. The maximum level of the immune reply to the injection of the majority of the antigens is reached after 40-60 days from the first injection. After the first cycle of the immunization of the rabbits 30 days are provided to restore their health and repeat the immunization that includes 1-3 intravenous injections. In order to obtain the antiserum the blood is gathered from the immunized rabbits to the centrifuge tube of 50 ml volume. With the means of the wooden spatula the formed clots are eliminated from the walls of the tube and the stick is placed to the clot that is formed at the centre of the tube. The blood is placed to refrigerator (the temperature is 4°C) for a night. The next day the clot that attached to the spatula is eliminated and the remaining liquid is centrifuged under 13000g during 10 minutes. The supernatant (over-sediment liquid) represents antiserum. The obtained antiserum must have yellow color. 20% NaN₃ may be added till the final concentration of 0,02% and it should be kept frozen under the temperature of -20°C (or without the addition of NaN₃ under the temperature of -70°) before use. The separation of the antibodies from the antiserum to the tumor necrosis factor - alpha can be represented in the following way:
   1. 10 ml of rabbit antiserum is diluted in 2 times 0,15 M of NaCl, add 6,26 g Na₂SO₄, then you should mix and incubate for 12-16 hours under the temperature of 4°C;
   2. The formed sediment is eliminated by the means of centrifuging, diluted in 10 ml of phosphate buffer and then dialyzed to the same buffer during the night under the room temperature;
   3. After the elimination of the sediment by the means of centrifuge the solution is planted to the column with DEAE-cellulose that is balanced with the phosphate buffer;
   4. The fraction of the antibodies is defined by the measurement of the optical density of the eluate under 280 nm.

The cleaning of the antibodies is executed by the method of affine chromatography at the column with antigen by the means of bounding the antibodies to the tumor necrosis factor alpha with antigen (tumor necrosis factor alpha), connected to the insoluble matrix of the column with the following elution of the antibodies by the concentrated solutions of salt. The buffer solution of polyclonal antibodies to the tumor necrosis factor alpha obtained by this method which has the concentration of 0,5 ÷ 5,0 mg/ml, preferably 2,0 ÷ 3,0 mg/ml is used as matrix (initial) solution for the further preparation of activated-potentiated form of the antibodies.

The polyclonal antibodies to the human alpha-interferone are obtained by the means of the method that was stated above; the role of immunogene (antigen) for the rabbit immunization is represented by the adjuvant and the entire molecule of human alpha-interferone of one of the following sequences:
Human alpha-interferone (subtype 2):
Human alpha-interferone (subtype 1/13): 
MASPFALLMV LVVLSCKSSC SLGCDLPETH SLDNRRTLML
LAQMSRISPS SCLMDRHDFG
FPQEEFDGNQ FQKAPAISVL HELIQQIFNL FTTKDSSAAW
DEDLLDKFCT ELYQQLNDLE
ACVMQEERVG ETPLMNADSI LAVKKYFRRI TLYLTEKKYS
PCAWEVVRAE IMRSLSLSTN
LQERLRRKE
Human alpha-interferone (subtype 17): 
MALSFSLLMA VLVLSYKSIC SLGCDLPQTH SLGNRRALIL
LAQMGRISPF SCLKDRHDFG
LPQEEFDGNQ FQKTQAISVL HEMIQQTFNL FSTEDSSAAW
EQSLLEKFST ELYQQLNNLE
ACVIQEVGME ETPLMNEDSI LAVRKYFQRI TLYLTEKKYS
PCAWEVVRAE IMRSLSFSTN
 LQKILRRKD
Human alpha-interferone (subtype 4): 
MALSFSLLMA VLVLSYKSIC SLGCDLPQTH SLGNRRALIL
LAQMGRISHF SCLKDRHDFG
FPEEEFDGHQ FQKAQAISVL HEMIQQTFNL FSTEDSSAAW
EQSLLEKFST ELYQQLNDLE
ACVIQEVGVE ETPLMNEDSI LAVRKYFQRI TLYLTEKKYS
PCAWEVVRAE IMRSLSFSTN
LQKRLRRKD
Human alpha-interferone (subtype 8): 
MALTFYLLVA LVVLSYKSFS SLGCDLPQTH SLGNRRALIL
LAQMRRISPF SCLKDRHDFE
FPQEEFDDKQ FQKAQAISVL HEMIQQTFNL FSTKDSSAAL
DETLLDEFYI ELDQQLNDLE
SCVMQEVGVI ESPLMYEDSI LAVRKYFQRI TLYLTEKKYS
SCAWEVVRAE IMRSFSLSIN
LQKRLKSKE
Human alpha-interferone (subtype 7): 
MARSFSLLMV VLVLSYKSIC SLGCDLPQTH SLRNRRALIL
LAQMGRISPF SCLKDRHEFR
FPEEEFDGHQ FQKTQAISVL HEMIQQTFNL FSTEDSSAAW
EQSLLEKFST ELYQQLNDLE
ACVIQEVGVE ETPLMNEDFI LAVRKYFQRI TLYLMEKKYS
PCAWEVVRAE IMRSFSFSTN
LKKGLRRKD
Human alpha-interferone (subtype 21): 
MALSFSLLMA VLVLSYKSIC SLGCDLPQTH SLGNRRALIL
LAQMGRISPF SCLKDRHDFG
FPQEEFDGNQ FQKAQAISVL HEMIQQTFNL FSTKDSSATW
EQSLLEKFST ELNQQLNDLE
 
ACVIQEVGVE ETPLMNVDSI LAVKKYFQRI TLYLTEKKYS
PCAWEVVRAE IMRSFSLSKI
FQERLRRKE
Human alpha-interferone (subtype 10): 
MALSFSLLMA VLVLSYKSIC SLGCDLPQTH SLGNRRALIL
LGQMGRISPF SCLKDRHDFR
IPQEEFDGNQ FQKAQAISVL HEMIQQTFNL FSTEDSSAAW
EQSLLEKFST ELYQQLNDLE
ACVIQEVGVE ETPLMNEDSI LAVRKYFQRI TLYLIERKYS
PCAWEVVRAE IMRSLSFSTN
LQKRLRRKD
Human alpha-interferone (subtype 14): 
MALPFALMMA LVVLSCKSSC SLGCNLSQTH SLNNRRTLML
MAQMRRISPF SCLKDRHDFE
FPQEEFDGNQ FQKAQAISVL HEMMQQTFNL FSTKNSSAAW
DETLLEKFYI ELFQQMNDLE
ACVIQEVGVE ETPLMNEDSI LAVKKYFQRI TLYLMEKKYS
PCAWEVVRAE IMRSLSFSTN
LQKRLRRKD
Human alpha-interferone (subtype 5):

MALPFVLLMA LVVLNCKSIC SLGCDLPQTH SLSNRRTLMI
MAQMGRISPF SCLKDRHDFG
FPQEEFDGNQ FQKAQAISVL HEMIQQTFNL FSTKDSSATW
DETLLDKFYT ELYQQLNDLE
ACMMQEVGVE DTPLMNVDSI LTVRKYFQRI TLYLTEKKYS
PCAWEVVRAE IMRSFSLSAN
LQERLRRKE

The usage of adjuvant and, for example, one of polypeptide fragments of human alpha-interferone is possible in order to obtain polyclonal bodies to human alpha-interferone as the immunogene (antigen) for the immunization of rabbits.

Polyclonal antibodies to CD8 receptor are obtained following the method stated above using adjuvant and entire molecule of CD8 receptor with the following amino-acidic sequence as the immunogen (antigen) for rabbit immunization:

1 MALVPVTALLL PLALLLHAAR PSQFRVSPLD RTWNLGETVE
LKCQVLLSNP TSGCSWLFQP

61 RGAAASPTFL LYLSQNKPKA AEGLDTQRFS GKRLGDTFVL
TLSDFRRENE GYYFCSALSN

121 SIMYFSHFVP VFLPAKPTTT PAPRPPTPAP TIASQPLSLR
PEACRPAAGG AVHTRGLDFA

181 CDIYIWAPLA GTCGVLLLSL VITLYCNHRN RRRVCKCPRP
VVKSGDKPSL SARYV.

The usage of adjuvant and, for example, one of the fragments of CD8 receptor selected from the following sequences is possible in order to obtain polyclonal bodies to CD8 receptor as the immunogen (antigen) for the immunization of rabbits:
11-30:
   PLALLLHAAR PSQFRVSPLD;
81-100:
   AEGLDTQRFS GKRLGDTFVL;
121-140:
   SIMYFSHFVP VFLPAKPTTT;
201-210:
   VITLYCNHRN;
221-235:
   WKSGDKPSL SARYV

In order to prepare the medicinal agent the usage of three water-alcohol solutions of primary matrix solution of the antibodies is preferred, which are correspondently diluted in 100¹², 100³⁰ and 100²⁰⁰ times which corresponds to the centesimal solutions of C 12, C30 and C 200 prepared by the homeopathic technology. During the execution of the claimed medicinal agent in solid medicine form the mixture of the indicated components is put on the neutral carrier.

The activated-potentiated form of each component is prepared by the way of the uniform decrease of the concentration as the result of solution of 1 part of each solution that is to be diluted starting with the mentioned matrix solution, in 9 parts (for the decimal solution D) or 99 parts (for centesimal solution C) or 999 parts (for millesimal solution M) of the neutral solution in combination with multiple vertical shaking (potentiation or "dinamization") of each produced solution and the usage of separate containers for every further solution till the production of the required potency - the multiplicity of the solution by the homeopathic method (see, for example, W. Shwabe "Homeopathic medicinal preparations", M., 1967, p. 14-29).
The outer treatment during the process of concentration decrease also may be executed by the use of ultrasound, electromagnetic or other physical action.

For example, for the preparation of 12^{th} centesimal solution of C12 one part of the matrix (primary) solution of antibodies, for example, to the tumor necrosis factor-alpha with the concentration of 2,5 mg/ml is solved in 99 parts of neutral water or hydroalcoholic solvent and shaken vertically many times (10 and more times) - they potentiate, producing first centesimal C1 solution. From the 1^{st} centesimal solution of C1 the 2^{nd} centesimal solution of C2 is produced. The given operation is repeated for 11 times, producing 12^{th} centesimal solution of C12. In this way, the 12^{th} centesimal solution of C 12 represents the solution that is produced by consequently diluting the 1^{st} part of the primary matrix solution for 12 times in different containers, which is the solution of antibodies to human gamma-interferone with the concentration of 2,5 mg/ml in 99 parts of the neutral solvent, i.e. the solution that is produced by diluting the matrix solution in 100¹² times. Analogical operations with the correspondent dilution multiplicity are executed in order to produce C30 and C50.

When using, for example, activated-potentiated form of antibodies to the tumor necrosis factor-alpha in the form of the mixture of different, predominantly centesimal, solutions, each solution (for example, C12, C30, C50) is prepared separately using the technology described above till the solution which is 3 solutions less than the final (correspondently, till the producing of C9, C27, C47) and then 1 part of each component to one container is brought in correspondence to the composition of the mixture and mixed with the required amount of the solvent (with 97 parts for the centesimal solution correspondently). Further, the produced mixture is consequently diluted for two times in the proportion of 1 to 100, potentiating the produced solution after each solving operation. Meanwhile, the activated-potentiated form of antibodies is produced, for example, to human gamma-interferone in ultra-low dosage obtained by diluting the matrix solution in 100¹², 100³⁰, 100⁵⁰ times, equivalent to the mixture of the centesimal solutions C12, C30, C50.

The use of each component separately is possible in the form of the mixture of other different solutions, for example, decimal and/or centesimal, (D 20, C 30, C 100 or C12, C30, C200 etc.), prepared with the use of homeopathic technology, whose effectiveness is defined experimentally.

In order to produce the solid oral form of the claimed medicinal agent the watering process is executed in installation of boiling layer (for example, "Hüttlin Pilotlab" type produced by the company of Hüttlin Gmbh) till the saturation of the granules of the neutral substance which are injected in the pseudo-fluidized boiling layer; the substance is lactose (milk sugar) with the particle dimensions of 50 ÷ 500 µm, previously produced by the water or hydroalcoholic solution of activated-potentiated form of antibodies to CD4 receptor, predominantly, in the proportion of 1 kg of antibodies solution to 5 or 10 kg of lactose (1:5 - 1:10) with the simultaneous drying in the flow of the heated air that is directed under the grill under the temperature of not higher than 40°C. The calculated amount of lactose (10÷91% of the tablet mass) which is activated-potentiated with the antibodies form accordingly to the technology stated above, is loaded to the mixer and is mixed with lactose which is wetted with the activated-potentiated form of the antibodies, with the amounts of 3÷10% of the tablets mass and with pure lactose in the quantity of not more than 84% from the tablet mass (in order to decrease the cost and to simplify and accelerate the technological process without the decrease in medical action effectiveness). Then the microcrystalline cellulose in the amount of 5 ÷ 10% of the tablet mass and magnesium stearate in the amount of 1% of the tablet mass are added. The produced tablet mass is uniformly mixed and tableted by dry pressing (for example, in the tablet-press Korsch - XL 400). After the tabletation, we get the tablets of 300 mg that are saturated with the hydroalcoholic solution of the activated-potentiated form of antibodies to the tumor necrosis factor-alpha in ultra-low dosage of every component which is prepared from the matrix solution which is diluted in 100¹², 100³⁰, 100⁵⁰ which is equivalent to the mixture of the centesimal solutions of C12, C30 and C50, prepared accordingly to the homeopathic technology.

It is recommended to preferably use 1-2 tablets of the claimed medicinal agent 2-4 times per day.

### Example 1.

Antiretroviral action of the claimed medicinal agent was researched by inhibiting the replication of HIV in the culture of mononuclear cells of the peripheral human blood which were infected *in vitro* by the strain of HIV-1-LAI. The effectiveness of the HIV replication inhibition was estimated accordingly to the content of the basic nucleocapsid protein r24 HIV in supernatant.

For the execution of experimental researches were used affine purified rabbit polyclonal antibodies to the tumor necrosis factor alpha, that were prepared accordingly to the order by the specialized biotechnological company and that was the base for the preparation of activated-potentiated form of the water solutions of the antibodies to the tumor necrosis factor alpha in ultra-low dosage that were produced using the homeopathic technology following over-dilution of the primary matrix solution (concentration of 2,5 mg/ml) in 100¹², 100³⁰, 100⁵⁰ times, equivalent to the mixture of centesimal homeopathic solutions of C12, C30, C50 (further - ESD AB to TNF alpha). The estimation of the antiviral activity of the complex preparation was held with the use of mononuclear cells of human peripheral blood that were infected *in vitro* by the strain of HIV-1-LAI.

Mononuclears of the human peripheral blood were separated from the blood of healthy antibody-negative donors with the use of centrifuge in the gradient of density of Ficoll-Hypaque. The cells activated during 3 days with the usage of 1mcg/ml of fitogemmaglutine P and 5 ME/ml human recombinant interleukin.

For the estimation of antiretroviral activity the preparations were spilled to the cup that contained 100 µL of the activated mononuclears of the human peripheral blood, 24 hours previously or 15 minutes after the cells were infected by the strain of HIV-1-LAI in the dosage of 100 TCID50 (50 µL of the inoculum of the HIV-1-LAI). Prior to placing to the cup, ESD AB to TNF alpha (12,5 µL) or azydothimidine (active substance - zidovudine) in the dosage of 1000 nM (the comparison preparation) was mixed with the medium of RPMI1640 (DIFCO) until reaching the final volume of 50 µL.
Supernatants of the cells culture were gathered on the 7^{th} day after the infection. The effectiveness of the preparations was defined from the inhibition of HIV replication, that was estimated by the content of the basic nucleocapsid protein r24 HIV in the supernatants of the cells using the method of EIA (Retrotek Elisa kit).

It is shown that ESD AB to TNF alpha inhibit the replication of HIV by 92 ± 3% when placed to the cup 24 hours previously and by 13 ± 13% when placed to the cup 15 minutes after the cells are infected by the strain of HIV-1-LAI correspondently. Azidothymidine in the dosage of 1000 nM inhibited the replication of HIV by 99 ± 0 and 99 ± 1% when placed to the cup 24 hours previously and 15 minutes after the cells were infected by the strain of HIV-1-LAI correspondently.

So, during the research *in vitro* the high antiretroviral activity of the claimed agent at the base of activated - potentiated form of the rabbit polyclonal antibodies to TNF alpha is showed.

Note: TCID50 is the dosage that infects 50% of the cells from the tissue culture.

### Example 2.

Antiretroviral action of the claimed medicinal agent was inspected inhibiting the replication of HIV in the culture of the mononuclear cells of human peripheral blood that were infected *in vitro* by the strain of HIV-1-LAI. The effectiveness of inhibiting the replication of HIV was estimated by the content of the basic nucleocapsid protein of r24 HIV in supernatants.
In order to execute the experimental researches were used affine purified rabbit polyclonal antibodies to CD8 that were prepared accordingly to the order by the specialized biotechnological company and they were used as the base for the preparation of activated-potentiated form of the water solution of antibodies to CD8 in ultra-low dosage that were produced using the homeopathic technology by the over-dilution of the primary matrix solution (concentration of 2,5 mg/ml) in 100¹², 100³⁰, 100⁵⁰ times that are equivalent to the mixture of the centesimal homeopathic dilutions of C12, C30, C50 (onwards - ESD AB to CD8).

The estimation of the antiviral activity of the complex preparation was presented with the use of mononuclear cells of the human peripheral blood infected *in vitro* by the strain of HIV-1-LAI.
Mononuclears of human peripheral body were separated from the blood of healthy serum-negative donors with the help of centrifugation in the density gradient of Ficoll-Hypaque. The cells activated during 3 days with the use of 1 mcg/ml of phytohemaglyutin P and 5 ME/ml of the human recombinant interleukin-2.

For the estimation of the antiretroviral activity the preparations were placed into the cup that contained 100 µL of the activated mononuclears of the human peripheral blood, 24 hours previously and 15 minutes after the infection of the cells by the strain of HIV-1-LAI in the dosage of 100 TCID50 (50 µL of the inoculum of the strain of HIV-1-LAI). Previously to placing to the cup ESD AB to CD8 (12,5 µL) or azidothymidine (active substance - zidovudine) in the dosage of 1000 nM (comparison preparation) was mixed with the medium of RPMI1640 (DIFCO) until the final volume of 50 µL was reached.

Supernatants of the cell cultures were gathered on the 7^{th} day after the infection. The preparations effectiveness was defined by the inhibition of HIV replication which was estimated basing on the content of the basic nucleocapsid protein r24 HIV in the supernatants of the cells by the method of EIA (Retrotek Elisa kit).

It is shown that ESD AB to CD8 inhibit the replication of HIV by 87 ± 11 % when placed to the cup 24 hours previously and by 40 ± 4% when placed to the cap 15 minutes after the infection of the cells by the strain of HIV-1-LAI correspondently. Azydothimidine in the dosage of 1000 nM inhibited the replication of HIV by 99 ± 0 and 99 ± 1% when placed to the cup 24 hours previously and 15 minutes after the infection of the cells by the strain of HIV-1-LAI correspondently.

Thus, in the *in vitro* research the high antiretroviral activity of the ultra-low dosages of the rabbit polyclonal antibodies to CD8 is shown.

Note: TCID50 - dosages, infecting 50% of the cells of culture tissue.

### Example 3

The estimation of the antiretroviral activity of the activated-potentiated forms of the water dilutions of the polyclonal affine purified rabbit antibodies to interferon alpha in ultra-low dosages (ULD) produced by the over-dilution of the initial matrix solution (concentration of 2,5 mg/ml) in 100¹², 100³⁰, 100⁵⁰ times, equivalent to the mixture of the centesimal homeopathic dilutions of C12, C30, C50 (ESD AB to IFN - α) was executed using the mononuclear cells of the human peripheral blood, that were infected *in vitro* by the strain of HIV-1-LAI. Azydothimidine was used as the comparison preparation.

Mononuclears of the human peripheral body were separated from the blood of healthy serum-negative donors with the help of centrifugation in the density gradient of Ficoll-Hypaque. The cells activated during 3 days with the use of 1 mcg/ml of phytogemmaglyutin P and 5 ME/ml of human recombinant interleukin-2.
The cells were infected by the strain of HIV-1-LAI, placing 50 µL of the inoculum of the strain of HIV-1-LAI that corresponds to the dosage of 100 TCID50 (dosage, infecting 50% of the cells of culture tissue).

For the estimation of the retroviral activity, the preparation ASD AB to IFN-α and the comparison preparation of azydothimidine were placed to the cup which contained 100 µL of the activated mononuclears of the human peripheral blood, 24 hours previously and 15 minutes after the cells infection by the strain of HIV-1-LAI. Previously to placing to the cups, the preparation of ESD AB to IFN-α (12,5 µL) and the preparation of azydothimidine in the dosage of 1000 nM were mixed with the medium of RPMI1640 (DIFCO) until the final volume of 50 µL was reached.

Supernatants of the cells culture were collected on the 7^{th} day after the infection. The preparations effectiveness was defined by the inhibition of the HIV replication that was estimated by the content of the basic nucleocapsid HIV protein r24 in the supernatants of the cells by the method of EIA (Retrotek Elisa kit).
It is shown that the preparation of ESD AB to IFN-α inhibits the replication of HIV by 95 ± 2% when placed to the cup 24 hours previously to the infection and 59 ± 14% when placed 15 minutes after the infection by the strain of HIV-1-LAI correspondently. Azydothimidine in the dosage of 1000 nM inhibits the replication of HIV by 99 ± 0 and 99 ± 1% when placed to the cup 24 hours previously and 15 minutes after the infection of the cells by the strain of HIV-1-LAI correspondently.

Thus, in the *in vitro* research the high antiretroviral activity of the preparation of ESD AB to IFN- α is showed.

## Claims

1. Medicinal agent for the prevention of HIV contamination, prevention and treatment of the diseases caused by HIV or HIV-associated diseases, including AIDS, that is **characterized by** the content of activated-potentiated form of antibodies to protein or peptide of the immune system that interacts with HIV or whose content and/or functional activity changes thanks to the HIV contamination.

2. Medicinal agent by p.1 that is characterized with the matter that the activated-potentiated form of antibodies to the solved antigen is used.

3. Medicinal agent by p. 2 that is characterized with the matter that antigen cytokines are used excluding gamma-interferon as the solved antigens.

4. Medicinal agent by p. 1 characterized with the matter that the activated-potentiated form of antibodies to the antigen which is connected to the outer membrane of the cells of the immune system is used.

5. Medicinal agent by p. 4 that is characterized with the matter that the immune-competent cells are used as the antigen that is connected to the outer membrane of the cells of the immune system.

6. Medicinal agent by p. 4 that is characterized with the matter that the differentiation clusters are used excluding the CD4 molecules of T-lymphocytes as the antigen which is connected to the outer membrane of the cells of immune system.

7. Medicinal agent by p. 2 or p. 4 that is characterized with the matter that the activated-potentiated form of the antibodies to the solved antigen or activated-potentiated form of the antibodies to the antigen which is connected to the outer membrane of the cells of the immune system are used as of activated-potentiated water or hydroalcoholic solution whose activity is conditioned by the process of consequent multiple dilution of the matrix-initial solution of the antibodies in water or hydroalcoholic solvent in combination with the outer mechanical action - vertical shaking of each dilution.

8. Medicinal agent by p. 2 or p. 4 that is characterized with the matter that it is produced in solid medical form in kind of pharmaceutical composition which contains technologically necessary amount of the neutral carrier which is saturated with the mixture of water or hydroalcoholic solutions of activated - potentiated form of antibodies to solved antigen or activated-potentiated form of antibodies to the antigen which is connected to the outer membrane of the cells of the immune system and pharmaceutically acceptable additives.

9. Medicinal agent by p. 2 or p. 4 that is characterized with the matter that the water or hydroalcoholic solutions of the activated - potentiated forms of antibodies to the solved antigen or the antigen that is connected to the outer membrane if the cells of the immune system, are produced by the means of multiple consequent dilution of the matrix - initial solution of antibodies in combination with outer mechanical action - vertical shaking after each dilution; meanwhile the concentration of the matrix solution is 0,5 ÷ 5,0 mg/ml.

10. Medicinal agent by p. 2 or p. 4 that is characterized with the matter that the activated - potentiated form of the antibodies is used in kind of the mixture of different, predominantly, centesimal dilutions by homeopathic technology.

11. Medicinal agent by p. 8 that is characterized with the matter that the pharmaceutically acceptable additives include lactose, microcrystalline cellulose and magnesium stearate.

12. The way of prevention of HIV contamination, prevention and treatment of the diseases caused by HIV or HIV-associated diseases including AIDS characterized with the matter that activated - potentiated form of antibodies to antigen - protein or peptide of immune system which interacts with the HIV or whose content and/or functional activity changes related to HIV contamination.

13. The method from p. 12, characterized with the matter that the activated-potentiated form of antibodies to the solved antigen or activated-potentiated form of antibodies to antigen connected to the outer membrane of the cells of immune system, are used as activated-potentiated water or hydroalcoholic solution whose activity is conditioned by the process of multiple dilution of the matrix - initial - solution of the antibodies in water or hydroalcoholic solvent in combination with the outer mechanical action - vertical shaking after each dilution.

14. The method from p. 13, characterized with the matter that the water or hydroalcoholic solutions of the activated-potentiated forms of antibodies to the solved antigen or the antigen that is connected to the outer membrane of the cells of the immune system are produced by the way of multiple consequent dilution of the matrix - initial - solution of the antibodies in combination with the outer mechanical action - vertical shaking of every dilution; meanwhile the concentration of the matrix solution is 0,5 ÷ 5,0 mg/ml.

15. The method from p. 12 which is characterized with the matter that the activated-potentiated form of antibodies to solved antigen is used.

16. The method from p. 12 which is characterized with the matter that cytokines are used as the solved antigen excluding gamma-interferon.

17. The method from p. 12 which is characterized with the matter that activated-potentiated form of the antibodies to antigen which is connected to the outer membrane of the cells of immune system is used.

18. The method from p. 12 that is characterized with the matter that as the antigen which is connected to the outer membrane of the immune system, the immune-competent cells are used.

19. The method from p. 12 that is characterized with the matter that the differentiation clusters are used excluding CD4 molecule of T-lymphocytes as the antigen which is connected to the outer membrane of the immune system cells.


SEQUENCE LISTING

<110> Epshtein et al.

<120> The drug and method for the prophylaxis of HIV infection
      and for the prophylaxis and treatment of diseases caused
      by or associated with HIV, including AIDS

<140> 11 814 861.8
<141> 2011-07-15

<150> RU2010133045
<151> 2010-08-06

<160> 28

<170> BiSSAP 1.0

<210> 1
<211> 233
<212> PRT
<213> Homo sapiens

<220>
<221> SOURCE
<222> 1..233
<223> /mol_type="protein"
      /organism="Homo sapiens"

<400> 1
Met Ser Thr Glu Ser Met Ile Arg Asp Val Glu Leu Ala Glu Glu Ala
1               5                   10                  15
Leu Pro Lys Lys Thr Gly Gly Pro Gln Gly Ser Arg Arg Cys Leu Phe
            20                  25                  30
Leu Ser Leu Phe Ser Phe Leu Ile Val Ala Gly Ala Thr Thr Leu Phe
        35                  40                  45
Cys Leu Leu His Phe Gly Val Ile Gly Pro Gln Arg Glu Glu Phe Pro
    50                  55                  60
Arg Asp Leu Ser Leu Ile Ser Pro Leu Ala Gln Ala Val Arg Ser Ser
65                  70                  75                  80
Ser Arg Thr Pro Ser Asp Lys Pro Val Ala His Val Val Ala Asn Pro
                85                  90                  95
Gln Ala Glu Gly Gln Leu Gln Trp Leu Asn Arg Arg Ala Asn Ala Leu
            100                 105                 110
Leu Ala Asn Gly Val Glu Leu Arg Asp Asn Gln Leu Val Val Pro Ser
        115                 120                 125
Glu Gly Leu Tyr Leu Ile Tyr Ser Gln Val Leu Phe Lys Gly Gln Gly
    130                 135                 140
Cys Pro Ser Thr His Val Leu Leu Thr His Thr Ile Ser Arg Ile Ala
145                 150                 155                 160
Val Ser Tyr Gln Thr Lys Val Asn Leu Leu Ser Ala Ile Lys Ser Pro
                165                 170                 175
Cys Gln Arg Glu Thr Pro Glu Gly Ala Glu Ala Lys Pro Trp Tyr Glu
            180                 185                 190
Pro Ile Tyr Leu Gly Gly Val Phe Gln Leu Glu Lys Gly Asp Arg Leu
        195                 200                 205
Ser Ala Glu Ile Asn Arg Pro Asp Tyr Leu Asp Phe Ala Glu Ser Gly
    210                 215                 220
Gln Val Tyr Phe Gly Ile Ile Ala Leu
225                 230


<210> 2
<211> 5
<212> PRT
<213> Homo sapiens

<220>

<221> SOURCE
<222> 1..5
<223> /mol_type="protein"
      /organism="Homo sapiens"

<400> 2
Pro Ser Asp Lys Pro
1               5


<210> 3
<211> 5
<212> PRT
<213> Homo sapiens

<220>
<221> SOURCE
<222> 1..5
<223> /mol_type="protein"
      /organism="Homo sapiens"

<400> 3
Val Ala Asn Pro Gln
1               5


<210> 4
<211> 135
<212> PRT
<213> Homo sapiens

<220>
<221> SOURCE
<222> 1..135
<223> /mol_type="protein"
      /organism="Homo sapiens"

<400> 4
Arg Asp Leu Ser Leu Ile Ser Pro Leu Ala Gln Ala Val Arg Ser Ser
1               5                   10                  15
Ser Arg Thr Pro Ser Asp Lys Pro Val Ala His Val Val Ala Asn Pro
            20                  25                  30
Gln Ala Glu Gly Gln Leu Gln Trp Leu Asn Arg Arg Ala Asn Ala Leu
        35                  40                  45
Leu Ala Asn Gly Val Glu Leu Arg Asp Asn Gln Leu Val Val Pro Ser
    50                  55                  60
Glu Gly Leu Tyr Leu Ile Tyr Ser Gln Val Leu Phe Lys Gly Gln Gly
65                  70                  75                  80
Cys Pro Ser Thr His Val Leu Leu Thr His Thr Ile Ser Arg Ile Ala
                85                  90                  95
Val Ser Tyr Gln Thr Lys Val Asn Leu Leu Ser Ala Ile Lys Ser Pro
            100                 105                 110
Cys Gln Arg Glu Thr Pro Glu Gly Ala Glu Ala Lys Pro Trp Tyr Glu
        115                 120                 125
Pro Ile Tyr Leu Gly Gly Val
    130                 135


<210> 5
<211> 17
<212> PRT
<213> Homo sapiens

<220>
<221> SOURCE
<222> 1..17
<223> /mol_type="protein"
      /organism="Homo sapiens"

<400> 5
Val Arg Ser Ser Ser Arg Thr Pro Ser Asp Lys Pro Val Ala His Val
1               5                   10                  15
Val


<210> 6
<211> 23
<212> PRT
<213> Homo sapiens

<220>
<221> SOURCE
<222> 1..23
<223> /mol_type="protein"
      /organism="Homo sapiens"

<400> 6
Phe Leu Ser Leu Phe Ser Phe Leu Ile Val Ala Gly Ala Thr Thr Leu
1               5                   10                  15
Phe Cys Leu Leu His Phe Gly
            20


<210> 7
<211> 18
<212> PRT
<213> Homo sapiens

<220>
<221> SOURCE
<222> 1..18
<223> /mol_type="protein"
      /organism="Homo sapiens"

<400> 7
Ile Gly Pro Gln Arg Glu Glu Phe Pro Arg Asp Leu Ser Leu Ile Ser
1               5                   10                  15
Pro Leu


<210> 8
<211> 38
<212> PRT
<213> Homo sapiens

<220>
<221> SOURCE
<222> 1..38
<223> /mol_type="protein"
      /organism="Homo sapiens"

<400> 8
Gln Leu Val Val Pro Ser Glu Gly Leu Tyr Leu Ile Tyr Ser Gln Val
1               5                   10                  15
Leu Phe Lys Gly Gln Gly Cys Pro Ser Thr His Val Leu Leu Thr His
            20                  25                  30
Thr Ile Ser Arg Ile Ala
        35


<210> 9
<211> 15
<212> PRT
<213> Homo sapiens

<220>
<221> SOURCE
<222> 1..15
<223> /mol_type="protein"
      /organism="Homo sapiens"

<400> 9
Pro Cys Gln Arg Glu Thr Pro Glu Gly Ala Glu Ala Lys Pro Trp
1               5                   10                  15

<210> 10
<211> 41
<212> PRT
<213> Homo sapiens

<220>
<221> SOURCE
<222> 1..41
<223> /mol_type="protein"
      /organism="Homo sapiens"

<400> 10
Ser Met Ile Arg Asp Val Glu Leu Ala Glu Glu Ala Leu Pro Lys Lys
1               5                   10                  15
Thr Gly Gly Pro Gln Gly Ser Arg Arg Cys Leu Phe Leu Ser Leu Phe
            20                  25                  30
Ser Phe Leu Ile Val Ala Gly Ala Thr
        35                  40

<210> 11
<211> 35
<212> PRT
<213> Homo sapiens

<220>
<221> SOURCE
<222> 1..35
<223> /mol_type="protein"
      /organism="Homo sapiens"

<400> 11
Val Leu Leu Thr His Thr Ile Ser Arg Ile Ala Val Ser Tyr Gln Thr
1               5                   10                  15
Lys Val Asn Leu Leu Ser Ala Ile Lys Ser Pro Cys Gln Arg Glu Thr
            20                  25                  30
Pro Glu Gly
        35

<210> 12
<211> 157
<212> PRT
<213> Homo sapiens

<220>
<221> SOURCE
<222> 1..157
<223> /mol_type="protein"
      /organism="Homo sapiens"

<400> 12

<210> 13
<211> 189
<212> PRT
<213> Homo sapiens

<220>
<221> SOURCE
<222> 1..189
<223> /mol_type="protein"
      /organism="Homo sapiens"

<400> 13
Met Ala Leu Thr Phe Tyr Leu Leu Val Ala Leu Val Val Leu Ser Tyr
1               5                   10                  15
Lys Ser Phe Ser Ser Leu Gly Cys Asp Leu Pro Gln Thr His Ser Leu
            20                  25                  30
Gly Asn Arg Arg Ala Leu Ile Leu Leu Ala Gln Met Arg Arg Ile Ser
        35                  40                  45
Pro Phe Ser Cys Leu Lys Asp Arg His Asp Phe Glu Phe Pro Gln Glu
    50                  55                  60
Glu Phe Asp Asp Lys Gln Phe Gln Lys Ala Gln Ala Ile Ser Val Leu
65                  70                  75                  80
His Glu Met Ile Gln Gln Thr Phe Asn Leu Phe Ser Thr Lys Asp Ser
                85                  90                  95
Ser Ala Ala Leu Asp Glu Thr Leu Leu Asp Glu Phe Tyr Ile Glu Leu
            100                 105                 110
Asp Gln Gln Leu Asn Asp Leu Glu Ser Cys Val Met Gln Glu Val Gly
        115                 120                 125
Val Ile Glu Ser Pro Leu Met Tyr Glu Asp Ser Ile Leu Ala Val Arg
    130                 135                 140
Lys Tyr Phe Gln Arg Ile Thr Leu Tyr Leu Thr Glu Lys Lys Tyr Ser
145                 150                 155                 160
Ser Cys Ala Trp Glu Val Val Arg Ala Glu Ile Met Arg Ser Phe Ser
                165                 170                 175
Leu Ser Ile Asn Leu Gln Lys Arg Leu Lys Ser Lys Glu
            180                 185

<210> 14
<211> 188
<212> PRT
<213> Homo sapiens

<220>
<221> SOURCE
<222> 1..188
<223> /mol_type="protein"
      /organism="Homo sapiens"

<400> 14

<210> 15
<211> 189
<212> PRT
<213> Homo sapiens

<220>
<221> SOURCE
<222> 1..189
<223> /mol_type="protein"
      /organism="Homo sapiens"

<400> 15
Met Ala Leu Ser Phe Ser Leu Leu Met Ala Val Leu Val Leu Ser Tyr
1               5                   10                  15
Lys Ser Ile Cys Ser Leu Gly Cys Asp Leu Pro Gln Thr His Ser Leu
            20                  25                  30
Gly Asn Arg Arg Ala Leu Ile Leu Leu Ala Gln Met Gly Arg Ile Ser
        35                  40                  45
Pro Phe Ser Cys Leu Lys Asp Arg His Asp Phe Gly Leu Pro Gln Glu
    50                  55                  60
Glu Phe Asp Gly Asn Gln Phe Gln Lys Thr Gln Ala Ile Ser Val Leu
65                  70                  75                  80
His Glu Met Ile Gln Gln Thr Phe Asn Leu Phe Ser Thr Glu Asp Ser
                85                  90                  95
Ser Ala Ala Trp Glu Gln Ser Leu Leu Glu Lys Phe Ser Thr Glu Leu
            100                 105                 110
Tyr Gln Gln Leu Asn Asn Leu Glu Ala Cys Val Ile Gln Glu Val Gly
        115                 120                 125
Met Glu Glu Thr Pro Leu Met Asn Glu Asp Ser Ile Leu Ala Val Arg
    130                 135                 140
Lys Tyr Phe Gln Arg Ile Thr Leu Tyr Leu Thr Glu Lys Lys Tyr Ser
145                 150                 155                 160
Pro Cys Ala Trp Glu Val Val Arg Ala Glu Ile Met Arg Ser Leu Ser
                165                 170                 175
Phe Ser Thr Asn Leu Gln Lys Ile Leu Arg Arg Lys Asp
            180                 185

<210> 16
<211> 189
<212> PRT
<213> Homo sapiens

<220>
<221> SOURCE
<222> 1..189
<223> /mol_type="protein"
      /organism="Homo sapiens"

<400> 16
Met Ala Leu Ser Phe Ser Leu Leu Met Ala Val Leu Val Leu Ser Tyr
1               5                   10                  15
Lys Ser Ile Cys Ser Leu Gly Cys Asp Leu Pro Gln Thr His Ser Leu
            20                  25                  30
Gly Asn Arg Arg Ala Leu Ile Leu Leu Ala Gln Met Gly Arg Ile Ser
        35                  40                  45
His Phe Ser Cys Leu Lys Asp Arg His Asp Phe Gly Phe Pro Glu Glu
    50                  55                  60
Glu Phe Asp Gly His Gln Phe Gln Lys Ala Gln Ala Ile Ser Val Leu
65                  70                  75                  80
His Glu Met Ile Gln Gln Thr Phe Asn Leu Phe Ser Thr Glu Asp Ser
                85                  90                  95
Ser Ala Ala Trp Glu Gln Ser Leu Leu Glu Lys Phe Ser Thr Glu Leu
            100                 105                 110
Tyr Gln Gln Leu Asn Asp Leu Glu Ala Cys Val Ile Gln Glu Val Gly
        115                 120                 125
Val Glu Glu Thr Pro Leu Met Asn Glu Asp Ser Ile Leu Ala Val Arg
    130                 135                 140
Lys Tyr Phe Gln Arg Ile Thr Leu Tyr Leu Thr Glu Lys Lys Tyr Ser
145                 150                 155                 160
Pro Cys Ala Trp Glu Val Val Arg Ala Glu Ile Met Arg Ser Leu Ser
                165                 170                 175
Phe Ser Thr Asn Leu Gln Lys Arg Leu Arg Arg Lys Asp
            180                 185



<210> 17
<211> 189
<212> PRT
<213> Homo sapiens

<220>
<221> SOURCE
<222> 1..189
<223> /mol_type="protein"
      /organism="Homo sapiens"

<400> 17
Met Ala Leu Ser Phe Ser Leu Leu Met Ala Val Leu Val Leu Ser Tyr
1               5                   10                  15
Lys Ser Ile Cys Ser Leu Gly Cys Asp Leu Pro Gln Thr His Ser Leu
            20                  25                  30
Gly Asn Arg Arg Ala Leu Ile Leu Leu Ala Gln Met Gly Arg Ile Ser
        35                  40                  45
Pro Phe Ser Cys Leu Lys Asp Arg His Asp Phe Gly Phe Pro Gln Glu
    50                  55                  60
Glu Phe Asp Gly Asn Gln Phe Gln Lys Ala Gln Ala Ile Ser Val Leu
65                  70                  75                  80
His Glu Met Ile Gln Gln Thr Phe Asn Leu Phe Ser Thr Lys Asp Ser
                85                  90                  95
Ser Ala Thr Trp Glu Gln Ser Leu Leu Glu Lys Phe Ser Thr Glu Leu
            100                 105                 110
Asn Gln Gln Leu Asn Asp Leu Glu Ala Cys Val Ile Gln Glu Val Gly
        115                 120                 125
Val Glu Glu Thr Pro Leu Met Asn Val Asp Ser Ile Leu Ala Val Lys

    130                 135                 140
Lys Tyr Phe Gln Arg Ile Thr Leu Tyr Leu Thr Glu Lys Lys Tyr Ser
145                 150                 155                 160
Pro Cys Ala Trp Glu Val Val Arg Ala Glu Ile Met Arg Ser Phe Ser
                165                 170                 175
Leu Ser Lys Ile Phe Gln Glu Arg Leu Arg Arg Lys Glu
            180                 185

<210> 18
<211> 189
<212> PRT
<213> Homo sapiens

<220>
<221> SOURCE
<222> 1..189
<223> /mol_type="protein"
      /organism="Homo sapiens"

<400> 18
Met Ala Ser Pro Phe Ala Leu Leu Met Val Leu Val Val Leu Ser Cys
1               5                   10                  15
Lys Ser Ser Cys Ser Leu Gly Cys Asp Leu Pro Glu Thr His Ser Leu
            20                  25                  30
Asp Asn Arg Arg Thr Leu Met Leu Leu Ala Gln Met Ser Arg Ile Ser
        35                  40                  45
Pro Ser Ser Cys Leu Met Asp Arg His Asp Phe Gly Phe Pro Gln Glu
    50                  55                  60
Glu Phe Asp Gly Asn Gln Phe Gln Lys Ala Pro Ala Ile Ser Val Leu
65                  70                  75                  80
His Glu Leu Ile Gln Gln Ile Phe Asn Leu Phe Thr Thr Lys Asp Ser
                85                  90                  95
Ser Ala Ala Trp Asp Glu Asp Leu Leu Asp Lys Phe Cys Thr Glu Leu
            100                 105                 110
Tyr Gln Gln Leu Asn Asp Leu Glu Ala Cys Val Met Gln Glu Glu Arg
        115                 120                 125
Val Gly Glu Thr Pro Leu Met Asn Ala Asp Ser Ile Leu Ala Val Lys
    130                 135                 140
Lys Tyr Phe Arg Arg Ile Thr Leu Tyr Leu Thr Glu Lys Lys Tyr Ser
145                 150                 155                 160
Pro Cys Ala Trp Glu Val Val Arg Ala Glu Ile Met Arg Ser Leu Ser
                165                 170                 175
Leu Ser Thr Asn Leu Gln Glu Arg Leu Arg Arg Lys Glu
            180                 185

<210> 19
<211> 189
<212> PRT
<213> Homo sapiens

<220>
<221> SOURCE
<222> 1..189
<223> /mol_type="protein"
      /organism="Homo sapiens"

<400> 19
Met Ala Leu Ser Phe Ser Leu Leu Met Ala Val Leu Val Leu Ser Tyr
1               5                   10                  15
Lys Ser Ile Cys Ser Leu Gly Cys Asp Leu Pro Gln Thr His Ser Leu
            20                  25                  30
Gly Asn Arg Arg Ala Leu Ile Leu Leu Gly Gln Met Gly Arg Ile Ser
        35                  40                  45
Pro Phe Ser Cys Leu Lys Asp Arg His Asp Phe Arg Ile Pro Gln Glu

    50                  55                  60
Glu Phe Asp Gly Asn Gln Phe Gln Lys Ala Gln Ala Ile Ser Val Leu
65                  70                  75                  80
His Glu Met Ile Gln Gln Thr Phe Asn Leu Phe Ser Thr Glu Asp Ser
                85                  90                  95
Ser Ala Ala Trp Glu Gln Ser Leu Leu Glu Lys Phe Ser Thr Glu Leu
            100                 105                 110
Tyr Gln Gln Leu Asn Asp Leu Glu Ala Cys Val Ile Gln Glu Val Gly
        115                 120                 125
Val Glu Glu Thr Pro Leu Met Asn Glu Asp Ser Ile Leu Ala Val Arg
    130                 135                 140
Lys Tyr Phe Gln Arg Ile Thr Leu Tyr Leu Ile Glu Arg Lys Tyr Ser
145                 150                 155                 160
Pro Cys Ala Trp Glu Val Val Arg Ala Glu Ile Met Arg Ser Leu Ser
                165                 170                 175
Phe Ser Thr Asn Leu Gln Lys Arg Leu Arg Arg Lys Asp
            180                 185

<210> 20
<211> 189
<212> PRT
<213> Homo sapiens

<220>
<221> SOURCE
<222> 1..189
<223> /mol_type="protein"
      /organism="Homo sapiens"

<400> 20
Met Ala Leu Pro Phe Val Leu Leu Met Ala Leu Val Val Leu Asn Cys
1               5                   10                  15
Lys Ser Ile Cys Ser Leu Gly Cys Asp Leu Pro Gln Thr His Ser Leu
            20                  25                  30
Ser Asn Arg Arg Thr Leu Met Ile Met Ala Gln Met Gly Arg Ile Ser
        35                  40                  45
Pro Phe Ser Cys Leu Lys Asp Arg His Asp Phe Gly Phe Pro Gln Glu
    50                  55                  60
Glu Phe Asp Gly Asn Gln Phe Gln Lys Ala Gln Ala Ile Ser Val Leu
65                  70                  75                  80
His Glu Met Ile Gln Gln Thr Phe Asn Leu Phe Ser Thr Lys Asp Ser
                85                  90                  95
Ser Ala Thr Trp Asp Glu Thr Leu Leu Asp Lys Phe Tyr Thr Glu Leu
            100                 105                 110
Tyr Gln Gln Leu Asn Asp Leu Glu Ala Cys Met Met Gln Glu Val Gly
        115                 120                 125
Val Glu Asp Thr Pro Leu Met Asn Val Asp Ser Ile Leu Thr Val Arg
    130                 135                 140
Lys Tyr Phe Gln Arg Ile Thr Leu Tyr Leu Thr Glu Lys Lys Tyr Ser
145                 150                 155                 160
Pro Cys Ala Trp Glu Val Val Arg Ala Glu Ile Met Arg Ser Phe Ser
                165                 170                 175
Leu Ser Ala Asn Leu Gln Glu Arg Leu Arg Arg Lys Glu
            180                 185

<210> 21
<211> 189
<212> PRT
<213> Homo sapiens

<220>
<221> SOURCE
<222> 1..189
<223> /mol_type="protein"

/organism="Homo sapiens"

<400> 21
Met Ala Arg Ser Phe Ser Leu Leu Met Val Val Leu Val Leu Ser Tyr
1               5                   10                  15
Lys Ser Ile Cys Ser Leu Gly Cys Asp Leu Pro Gln Thr His Ser Leu
            20                  25                  30
Arg Asn Arg Arg Ala Leu Ile Leu Leu Ala Gln Met Gly Arg Ile Ser
        35                  40                  45
Pro Phe Ser Cys Leu Lys Asp Arg His Glu Phe Arg Phe Pro Glu Glu
    50                  55                  60
Glu Phe Asp Gly His Gln Phe Gln Lys Thr Gln Ala Ile Ser Val Leu
65                  70                  75                  80
His Glu Met Ile Gln Gln Th Phe Asn Leu Phe Ser Thr Glu Asp Ser
                85                  90                  95
Ser Ala Ala Trp Glu Gln Ser Leu Leu Glu Lys Phe Ser Thr Glu Leu
            100                 105                 110
Tyr Gln Gln Leu Asn Asp Leu Glu Ala Cys Val Ile Gln Glu Val Gly
        115                 120                 125
Val Glu Glu Thr Pro Leu Met Asn Glu Asp Phe Ile Leu Ala Val Arg
    130                 135                 140
Lys Tyr Phe Gln Arg Ile Thr Leu Tyr Leu Met Glu Lys Lys Tyr Ser
145                 150                 155                 160
Pro Cys Ala Trp Glu Val Val Arg Ala Glu Ile Met Arg Ser Phe Ser
                165                 170                 175
Phe Ser Thr Asn Leu Lys Lys Gly Leu Arg Arg Lys Asp
            180                 185

<210> 22
<211> 189
<212> PRT
<213> Homo sapiens

<220>
<221> SOURCE
<222> 1..189
<223> /mol_type="protein"
      /organism="Homo sapiens"

<400> 22
Met Ala Leu Pro Phe Ala Leu Met Met Ala Leu Val Val Leu Ser Cys
1               5                   10                  15
Lys Ser Ser Cys Ser Leu Gly Cys Asn Leu Ser Gln Thr His Ser Leu
            20                  25                  30
Asn Asn Arg Arg Thr Leu Met Leu Met Ala Gln Met Arg Arg Ile Ser
        35                  40                  45
Pro Phe Ser Cys Leu Lys Asp Arg His Asp Phe Glu Phe Pro Gln Glu
    50                  55                  60
Glu Phe Asp Gly Asn Gln Phe Gln Lys Ala Gln Ala Ile Ser Val Leu
65                  70                  75                  80
His Glu Met Met Gln Gln Thr Phe Asn Leu Phe Ser Thr Lys Asn Ser
                85                  90                  95
Ser Ala Ala Trp Asp Glu Thr Leu Leu Glu Lys Phe Tyr Ile Glu Leu
            100                 105                 110
Phe Gln Gln Met Asn Asp Leu Glu Ala Cys Val Ile Gln Glu Val Gly
        115                 120                 125
Val Glu Glu Thr Pro Leu Met Asn Glu Asp Ser Ile Leu Ala Val Lys
    130                 135                 140
Lys Tyr Phe Gln Arg Ile Thr Leu Tyr Leu Met Glu Lys Lys Tyr Ser
145                 150                 155                 160
Pro Cys Ala Trp Glu Val Val Arg Ala Glu Ile Met Arg Ser Leu Ser
                165                 170                 175
Phe Ser Thr Asn Leu Gln Lys Arg Leu Arg Arg Lys Asp
            180                 185

<210> 23
<211> 235
<212> PRT
<213> Homo sapiens

<220>
<221> SOURCE
<222> 1..235
<223> /mol_type="protein"
      /organism="Homo sapiens"

<400> 23

<210> 24
<211> 20
<212> PRT
<213> Homo sapiens

<220>
<221> SOURCE
<222> 1..20
<223> /mol_type="protein"
      /organism="Homo sapiens"

<400> 24
Pro Leu Ala Leu Leu Leu His Ala Ala Arg Pro Ser Gln Phe Arg Val
1               5                   10                  15
Ser Pro Leu Asp
            20

<210> 25
<211> 20
<212> PRT
<213> Homo sapiens

<220>
<221> SOURCE
<222> 1..20
<223> /mol_type="protein"
      /organism="Homo sapiens"

<400> 25
Ala Glu Gly Leu Asp Thr Gln Arg Phe Ser Gly Lys Arg Leu Gly Asp
1               5                   10                  15
Thr Phe Val Leu
            20

<210> 26
<211> 20
<212> PRT
<213> Homo sapiens

<220>
<221> SOURCE
<222> 1..20
<223> /mol_type="protein"
      /organism="Homo sapiens"

<400> 26
Ser Ile Met Tyr Phe Ser His Phe Val Pro Val Phe Leu Pro Ala Lys
1               5                   10                  15
Pro Thr Thr Thr
            20

<210> 27
<211> 10
<212> PRT
<213> Homo sapiens

<220>
<221> SOURCE
<222> 1..10
<223> /mol_type="protein"
      /organism="Homo sapiens"

<400> 27
Val Ile Thr Leu Tyr Cys Asn His Arg Asn
1               5                   10

<210> 28
<211> 15
<212> PRT
<213> Homo sapiens

<220>
<221> SOURCE
<222> 1..15
<223> /mol_type="protein"
      /organism="Homo sapiens"

<400> 28
Val Val Lys Ser Gly Asp Lys Pro Ser Leu Ser Ala Arg Tyr Val
1               5                   10                  15